# FASCICULE DE BREVET EUROPEEN

(11) **EP 0 518 724 B1**
(45) Date de publication et mention de la délivrance du brevet: **13.11.1996**
(21) Numéro de dépôt: 92401489.7
(22) Date de dépôt: 01.06.1992
(51) Int. Cl.: C07D 339/08

(54) **Propénals substitués par un cycle dithiane et procédés de préparation de ces propénals**
Dithiansubstituierte Propenale und Verfahren zu ihrer Herstellung
Dithiane substituted propenals and process for their preparation

(30) Priorité: 14.06.1991 FR 9107293
(43) Date de publication de la demande: 16.12.1992
(73) Titulaire: L'OREAL, 75008 Paris (FR)
(72) Inventeur: Solladie, Guy, F-67000 Strasbourg (FR); Berl, Valérie, F-68200 Mulhouse (FR); Maignan, Jean, F-93290 Tremblay-les-Gonesse (FR)
(74) Mandataire: Peuscet, Jacques

(56) Documents cités:
- TETRAHEDRON LETTERS vol. 29, no. 18, 1988, GREAT BRITAIN pages 2197 - 2200; TOSHIO SATO ET AL.: 'Preparation of chiral C5-b
- CHEMISCHE BERICHTE vol. 115, no. 12, 3 Décembre 1982, WEINHEIM pages 3898 - 3903; KLAUS RUSTEMEIER ET AL.: '(E)-3-(1,3-Dithian-2-yl)acroleine als geschützte F
- Theodora W.Greene: "Protective Groups in Organic Synthesis", 1981, John Wiley and Sons, New-York*page 133, alinéa 2-page 134, alinéa 2;exemple 19*

## Description

La présente invention concerne des propénals substitués par un cycle dithiane en bout de chaîne ayant une structure stéréospécifique cis- ou trans- et des procédés de préparation de ces propénals permettant de les obtenir avec une bonne stéréospécificité.

Certains dithiane-propénals sont connus. C'est le cas, notamment, du dithianepropenal-trans de formule : 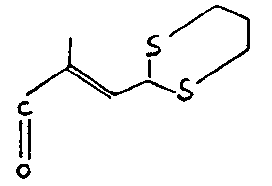 qui est décrit dans T. SAKO, K. HANAYAMA, T. FUJISAWA Tetrahedron Letters, 29, 2197 (1988) et K. RUSTEMEIER, E. BREITMALER Chem. Ber, 115, 3898 (1982).

La présente invention a donc pour premier objet les propénals de formule générale (I) : 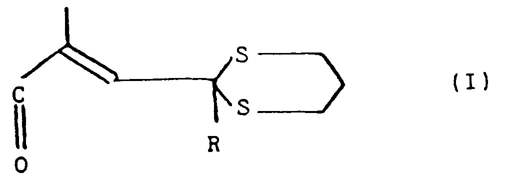 formule dans laquelle R est l'hydrogène ou un radical thioalkyle en C₁ - C₄ , le cycle dithiane étant en position cis par rapport à la fonction aldéhyde, lorsque R est H, et en position trans lorsque R est un radical thioalkyle.

Dans le présent texte et par convention, la position -trans du cycle dithiane est représentée en mettant l'axe de symétrie de ce cycle oblique vers le haut et la position -cis en orientant ledit axe vers le bas. Lorsque cet axe est représenté horizontal, la formule désigne indistinctement la forme stéréospécifique -cis ou la forme stéréospécifique -trans.

La présente invention a plus particulièrement pour objet le cis- propénal de formule (Ia) : 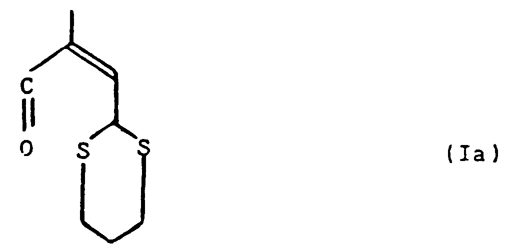 et le trans- propénal de formule (Ib) :

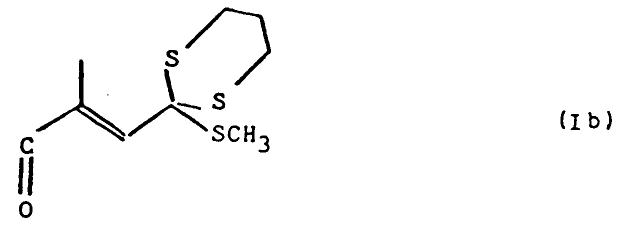

Ces composés peuvent être utilisés comme composés de départ dits "synthons" dans la synthèse de dérivés rétinoïdes stéréospécifiques substitués par un cycle dithiane de formule (II) : 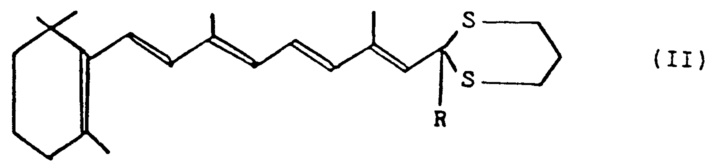 formule dans laquelle R a la même signification que pour la formule (I), le cycle dithiane étant en position cis lorsque R est H et en position trans lorsque R est un radical thioalkyle. Un procédé de préparation permettant d'obtenir, avec une bonne stéréospécificité, des rétinoïdes de formule (II) à partir des synthons de formule (I) est décrit dans EP-A-0 518 722, déposé à la même date que la présente demande et il est donné à titre d'illustration, dans les exemples 2 et 4 de la présente demande.

La présente invention a pour deuxième objet un procédé de préparation du dithiane propénal de formule (Ia) où le groupe dithiane est en position cis: 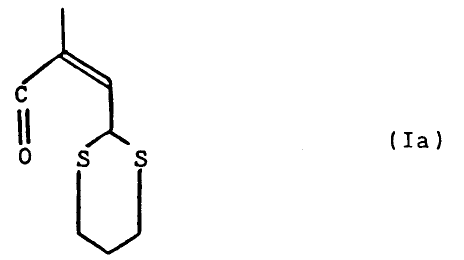 par réaction de β-formyl méthacrylate d'éthyle de structure cis et de formule : 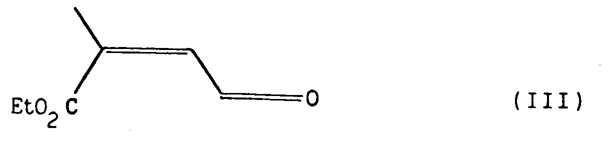 avec Et = éthyle
avec du 1,3-propane dithiol en présence de triflate de zinc, de façon à fixer un cycle dithiane sur le groupe formyle en position cis avec élimination d'eau et à obtenir le composé de formule : 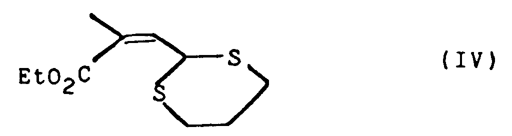 à réduire ce composé (IV) en présence d'hydrure de diisobutylaluminium pour obtenir le dithiane propénol de formule : 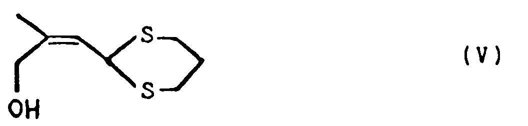 et à oxyder ledit propénol de formule (V) pour obtenir le cis- propénal de formule (Ia).

On a trouvé que les différentes réactions de préparation du dithiane-propénal cis de formule (Ia) sont stéréospécifiques et qu'à partir du β-formyl méthacrylate de méthyle de structure cis, on obtient un dithiane propénal, qui est également "cis", au moine de façon largement prépondérante.

La présente invention a pour troisième objet un procédé de préparation d'un propénal de formule (I), où le cycle dithiane est en position trans, dans lequel on fait réagir un alkylthiodithiane de formule: 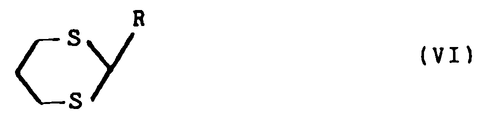 où R représente un radical thioalkyle en C₁- C₄, avec du n-butyl lithium puis avec de l'éthoxyméthacroléine de formule : 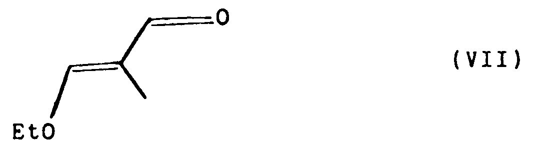 avec Et = éthyle
et on effectue une hydrolyse, de façon à obtenir le composé de formule : 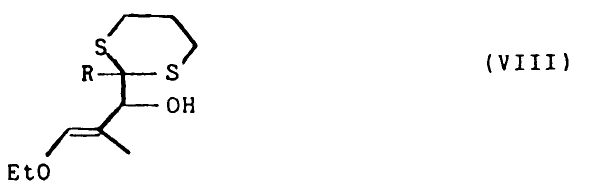 avec Et = Ethyle
après quoi on traite avec de l'acétone puis avec un acide pour obtenir ledit composé de formule (I) où le groupe dithiane est en position trans.

Les exemples ci-dessous donnés, à titre purement illustratifs et non limitatifs, permettront de mieux comprendre l'invention.

### EXEMPLE 1 - Préparation du composé de formule (Ia)

La réaction est effectuée selon la schéma suivant : 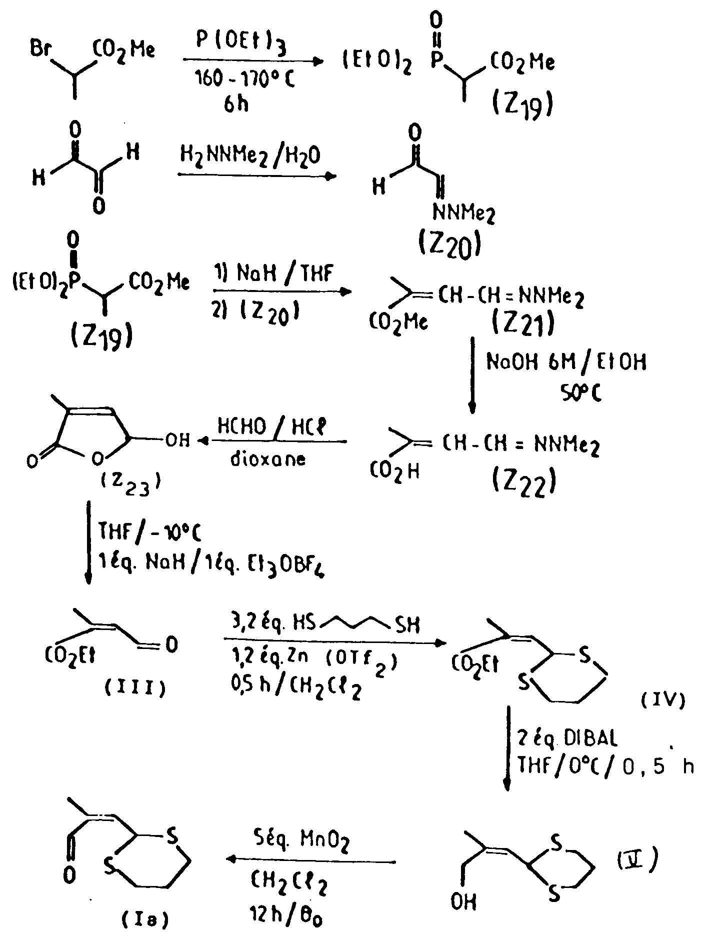 Dans ce schéma, les composés intermédiaires sont indiqués par les références (Zᵢ), i étant un nombre entier allant de 19 à 23. θₒ est la température ambiante.
1ère étape : Préparation du diéthyl phosphonopropionate de méthyle (Z₁₉ ) de formule : 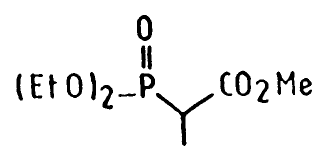
- avec: Me = méthyle
Et = éthyle

Un équivalent de 2-bromopropionate de méthyle (22,5 ml - 0,2 mole) est mélangé à 1,5 équivalent de triéthylphosphite (51,5 ml - 0,3 mole). Le milieu est chauffé progressivement à 160 - 170°C. Cette température est maintenue durant 6 heures. Au cours de la réaction, le bromure d'éthyle formé distille (température d'ébullition = 40°C) ainsi que de l'acrylate de méthyle (température d'ébullition = 80°C). Puis, l'excès de réactif est distillé sous vide (60 - 65°C, sous 66 Pascals). Le phosphonopropionate est distillé (75 - 80°C, sous 66 Pascals) afin d'obtenir 44,84 g d'un liquide incolore.
Le produit obtenu a les caractéristiques suivantes :
a) Chromatographie en couche mince CCM (hexane/acétate d'éthyle : 70/30) :
   Rf≃0.17
b) Masse moléculaire M = 224,2 g
c) La structure a été confirmée par résonance magnétique nucléaire(RMN ¹H et ¹³C) (200 MHz) (CDCl₃) et par étude du spectre infrarouge (IR) (CCl₄).

2ème étape : Préparation de la mono-N,N-diméthyl hydrazone du glyoxal (Z₂₀) de formule : 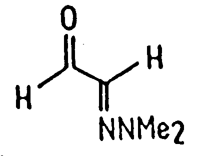 avec Me = méthyle
A 1,1 équivalent de glyoxal (0,5 mole - 29 ml de réactif en solution aqueuse à 30 %) dans de l'eau (300 ml) on ajoute goutte à goutte et sous agitation 1 équivalent de N,N-diméthylhydrazine (34,2 ml - 0,45 mole). Après 0,5 h à température ambiante le milieu est extrait au CH₂Cl₂(3 x 150 ml). Les phases organiques rassemblées sont séchées sur Na₂SO₄ , puis concentrées. Le liquide jaune clair (45 g) obtenu est utilisé tel quel.

Le produit obtenu a les caractéristiques suivantes : a) CCM (hexane/acétate d'éthyle : 70/30) :
   Rf≃0,23.
b) M = 100,12 g
c) La structure a été confirmée par RMN(¹H) (200 MHz) (CDCl₃) et par IR.

3ème étape : Préparation de N,N-diméthylhydrazone-4 méthyl-2 butène-2 oate de méthyle (Z₂₁) de formule : 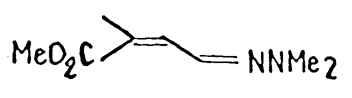 avec Me = méthyle
De l'hydrure de sodium (1,07 g - 0,0446 mole) est mis en suspension dans du tétrahydrofuranne (THF) anhydre (40 ml). Le milieu est refroidi à 0°C, puis 1 équivalent du phosphonoacétate (Z₁₉) est ajouté au goutte à goutte sous agitation (10 g - 0,0446 mole). Après 5 minutes, 1 équivalent de l'hydrazone (Z₂₀) (4,46 g - 0,0446 mole) est additionné lentement au milieu. Une gomme jaune brune se dépose rapidement au fond du ballon réactionnel. La réaction est suivie par chromatographie en couche mince jusqu'à disparition complète des produits de départ (15 à 30 minutes). Le milieu est alors hydrolysé par addition d'eau (10 ml), puis extrait à l'éther (3 x 50 ml). Les phases organiques rassemblées sont lavées par une solution saturée en NaCl (50 ml), séchées sur Na₂SO₄, puis concentrées. Le résidu est recristallisé dans un mélange acétone/pentane ; s'il est trop impur, une flash-chromatographie est nécessaire (éluant = hexane/acétate d'éthyle : 80/20 ; silice traitée avec 3 % de triéthylamine). On obtient 7,21 g de cristaux blancs.
Le rendement est de : 95 %
Les caractéristiques du produit obtenu sont les suivantes :
a) CCM (hexane/acétate d'éthyle : 70/30) :
RF≃0,56.
b) M = 170,2 g
c) Point de fusion Pf = 49-50°C
d) La structure a été confirmée par RMN(¹H et ¹³C) (200 MHz) (CDCl₃) et par IR.
e) L'analyse élémentaire donne les résultats suivants:

| % | Calculé | Trouvé |
|---|---|---|
| C | 56,46 | 56,28 |
| H | 8,29 | 8,40 |
| N | 16,46 | 16,40 |

4ème étape : Préparation de l'acide N,N-diméthylhydrazone-4 méthyl-2 butène-2 oïque (Z₂₂) de formule : 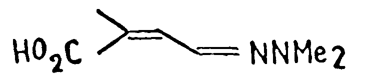 avec Me = méthyle
L'ester (Z₂₁) (10 g - 0,059 mole) est dissous dans un mélange 1/1 d'éthanol et de soude 6M (800 ml). Le mélange réactionnel est alors agité à 50°C jusqu'à disparition du produit de départ (30 min.). Il est ensuite dilué par addition d'eau (100 ml), et l'essentiel de l'alcool est évaporé soue vide. Le résidu est extrait une première fois à l'éther (2 x 100 ml) afin d'enlever tous les produits de dégradation organiques. Après une nouvelle addition d'éther (200 ml) à la phase aqueuse, celle-ci est acidifiée par addition lente d'acide sulfurique concentré en contrôlant le pH. Dès qu'un pH de 3 - 4 est atteint, le mélange réactionnel est extrait à l'éther (3 x 100 ml), séché sur Na₂SO₄, puis concentré. Le résidu est recristallisé dans un mélange acétone/pentane. On obtient 8,75 g de produit sous forme de longues aiguilles jaunes pâles.
Le rendement est de : 95 %
Les caractéristiques du produit obtenu sont les suivantes :
a) CCM (hexane/acétate d'éthyle : 70/30) :
Rf≃0,07.
b) Pf = 145-147°C
c) M = 156,19 g
d) La structure a été confirmée par RMN(¹H et ¹³C) (200 mHz) (CDCl₃) et par IR.
e) L'analyse élémentaire donne les résultats suivants:

| % | Calculé | Trouvé |
|---|---|---|
| C | 53,83 | 54,00 |
| H | 7,74 | 7,73 |
| N | 17,94 | 18,19 |

5ème étape : Préparation de la 5-hydroxy 3-méthyl 2 (5H)-furanone (Z₂₃) de formule :

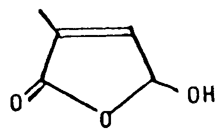

A l'hydrazone (Z₂₂) (6,5 g - 0,0416 mole) en solution dans du dioxane (130 ml), on ajoute 18 ml de formaldéhyde à 35 % dans l'eau et 13 ml d'acide chlorhydrique concentré. Le milieu est agité à température ambiante jusgu'à disparition du produit de départ (5 à 6 h). Puis, il est versé sur de la glace pilée, extrait au chlorure de méthylène (4 x 100 ml), séché sur Na₂SO₄, puis concentré.
Le buténolide obtenu est purifié en flash-chromatographie (éluant = hexane/acétate d'éthyle/éther : 40/30/30). On obtient 3,75 g de cristaux beiges.
Le rendement est de : 79 %
Les caractéristiques du produit obtenu sont les suivantes :
a) CCM (hexane/acétate d'éthyle : 50/50) :
RF≃0,29.
b) Pf = 70-72°C
c) M = 114,1 g
d) La structure a été confirmée par RMN (¹H et ¹³C)(200 MHz) (CDCl₃) et par IR.
e) L'analyse élémentaire donne les résultats suivants :

| % | Calculé | Trouvé |
|---|---|---|
| C | 52,63 | 52,82 |
| H | 5,30 | 5,33 |

6ème étape : Préparation du composé de formule (III) :
a) Préparation du tétrafluoroborate de triéthyloxonium (Et₃OBF₄).
A du trifluoroborure éthérate fraîchement distillé (30 ml - 0,243 mole) en solution dans de l'éther anhydre (60 ml) sous argon, est ajouté au goutte à goutte et sous forte agitation 0,75 équivalent d'épichlorhydrine (14,2 ml - 0,182 mole) de telle façon qu'il y ait reflux spontané. Ce reflux est maintenu durant 1 h, puis le milieu est laissé au repos durant la nuit. Un gel translucide se dépose au fond du ballon réactionnel. Il est filtré sous argon, lavé abondamment à l'éther anhydre, puis séché à la pompe à palettes. Un précipité blanc de 26 g de tétrafluoroborate de triéthyloxonium est obtenu. Il peut être conservé plusieurs jours au réfrigérateur sous argon.
Le rendement est de : 78 %
Le poids molaire est M = 183,94 g
b) Préparation du β-formyl méthacrylate d'éthyle sous forme cis de formule : 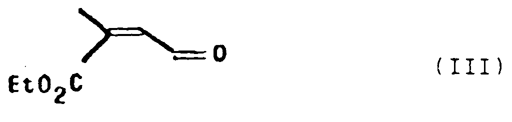 avec Et = éthyle
Le buténolide (Z ) (3 g - 0,026 mole) est solubilisé dans du chlorure de méthylène distillé (60 ml). Après refroidissement du milieu à -10°C, 1 équivalent d'hydrure de sodium (0,624 g - 0,026 mole) est additionné, suivi immédiatement d'1 équivalent de tétrafluoroborate de triéthyloxonium (4,97 g - 0,026 mole). Le milieu est agité pendant 1,5 h de -10°C à environ 5°C, avant d'être hydrolysé par addition d'un équivalent de triéthylamine (3,6 ml - 0,026 mole) et de 30 ml d'éther anhydre. Le précipité jaune pâle formé (complexe de la triéthylamine et du trifluoroborure) est filtré et lavé abondamment à l'éther anhydre. Le résidu est évaporé, puis traité par chromatographie sur colonne de silice (éluant = hexane/acétate d'éthyle : 80/20 ; silice traitée avec 3 % de triéthylamine). On obtient 2,22 g d'une huile jaune.
Le rendement est de : 60 %
Les caractéristiques du produit sont les suivantes :
a) CCM (hexane/acétate d'éthyle : 70/30) :
Rf≃0,62.
b) M = 142,16 g
c) La structure a été confirmée par RMN(¹H et¹³C) (200 MHz) (CDCl₃) et par IR.
d) L'analyse élémentaire donne les résultats suivants :

| % | Calculé | Trouvé |
|---|---|---|
| C | 59,14 | 58,88 |
| H | 7,09 | 7,21 |

7ème étape : Préparation du β-(1,3-dithiane) méthacrylate d'éthyle sous forme cis de formule : 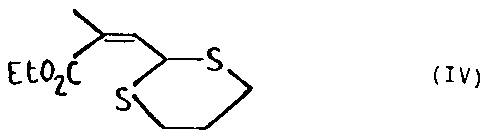 avec Et = éthyle
a) Préparation du triflate de zinc Zn(OTf)₂.
Un équivalent de carbonate de zinc (17,4 g - 0,139 mole) est mis en solution dans du méthanol sec (250 ml). A l'aide d'une ampoule à brome, 1,4 équivalent d'acide triflique (50 g - 0,195 mole) y sont ajoutés au goutte à goutte lent. La réaction est exothermique et un fort dégagement de CO₂ est observé. Le milieu est agité pendant 20 minutes à température ambiante, puis 2 h au reflux. Le méthanol est alors évaporé. Le résidu est séché par chauffage 2,5 h à 130°C sous 330 Pascals. On obtient 50,4 g d'une poudre blanche.
b) Préparation du composé de formule (IV) :
On met en solution 3,2 équivalents de 1,3-propane dithiol (4,85 ml - 0,048 mole) et 1,2 équivalent de Zn(OTf)₂ (6,54 g - 0,018 mole) dans du CH₂Cl₂ distillé (80 ml) sous argon. Après agitation du milieu pendant 15 minutes à température ambiante, 1 équivalent du composé de formule (III) (2,2 g - 0,015 mole) est additionné en solution dans du CH₂ Cl₂ (20 ml) au goutte à goutte rapide. La réaction est complète après 0,5 h et le milieu est dilué par addition d'eau (90 ml), puis d'une solution saturée en NH₄Cl jusqu'à ce que l'on atteigne un pH égal à 4 - 5 (environ 200 ml). La phase aqueuse est extraite par un mélange hexane/éther (1/1) (4 x 100 ml). Les phases organiques rassemblées sont lavées par une solution saturée en NH ₄ Cl (100 ml) afin de "casser" l'émulsion qui s'est formée, puis par de la soude 0,5M (3 x 100 ml), par une solution saturée en NaHCO₃ (100 ml), par de l'eau (2 x 100 ml) et enfin par une solution saturée en NaCl (100 ml). Après séchage sur Na₂SO₄ et évaporation du solvant, 3,6 g d'une huile incolore sont isolés.
Les caractéristiques du produit obtenu sont les suivantes :
a) CCM (hexane/acétate d'éthyle : 90/10) :
Rf≃0,36
b) M = 232,37 g.
c) La structure a été confirmée par RMN (¹H et ¹³C) (200 MHz) (CDCl₃) et par IR.
d) L'analyse élémentaire donne les résultats suivants :

| % | Calculé | Trouvé |
|---|---|---|
| C | 51,69 | 51,83 |
| H | 6,94 | 6,85 |

8ème étape : Préparation du composé (Z) 3-(1,3-dithiane 2-yl) 2-méthylpropénol de formule : 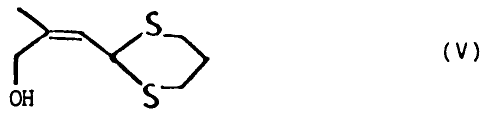
L'ester de formule (IV) (3,5 g - 0,015 mole) est mie en solution dans du tétrahydrofuranne (THF) anhydre (70 ml) sous argon. Après refroidissement du milieu à 0°C, 2,2 équivalents d'hydrure de diisobutylammonium (DIBAL) (33 ml de solution 1M dans le toluène) sont additionnes au goutte à goutte rapide. Après 0,5h la réaction est complète et le milieu est hydrolysé par addition lente de méthanol (1,55 ml). Le tout est verse sur un mélange d'acétate d'éthyle (600 ml) et d'une solution saturée en tartrate de sodium (74 ml). Une agitation vigoureuse de ce milieu durant 1 h permet de "casser" le gel qui s'est formé. La phase aqueuse est alors extraite à l'acétate d'éthyle (3 x 100 ml). Les phases organiques rassemblées sont lavées avec une solution saturée en NaCl (100 ml), sechées sur Na₂SO₄, puis concentrées. Le résidu est traite par chromatographie sur colonne de silice (éluant = hexane/acétate d'éthyle : 80/20 ; silice traitée avec 3 % de triéthylamine) pour donner 2,30 g d'une huile incolore.
Le rendement est de : 81 %
Le produit obtenu a les caractéristiques suivantes :
a) CCM (hexane/acétate d'éthyle : 70/30) :
Rf≃0,23.
b) La structure a été confirmée par RMN(¹H et ¹³C) (200 MHz) (CDCl₃) et par IR.
c) L'analyse élémentaire donne les résultats suivants :

| % | Calculé | Trouvé |
|---|---|---|
| C | 50,49 | 50,69 |
| H | 7,41 | 7,69 |

9ème étape : Préparation du (Z) 3-(1,3-dithiane 2-yl) 2-méthylpropénal de formule :

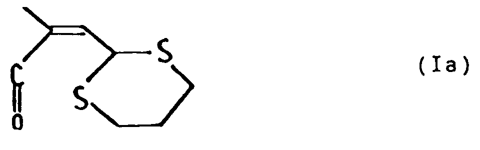

L'alcool de formule (V) (2,3 g - 0,012 mole) est dissous dans du CH ₂ Cl₂ distillé (150 ml) sous argon. Environ 5 équivalents de dioxyde de manganèse (5,25 g) sont ajoutés au milieu sous vigoureuse agitation. Après 4 h à température ambiante, la suspension est filtrée sur une petite couche de silice. La silice est abondamment lavée à l'acétate d'éthyle. Le filtrat est concentré sous vide. On obtient 2,15 g de cristaux beiges.
Le rendement est de : 95 %
Les caractéristiques du produit sont les suivantes :
a) CCM (hexane/acétate d'éthyle : 70/30) :
Rf≃0,52.
b) Pf = 102°C
c) M = 188,32 g
d) La structure a été confirmée par RMN (¹H et ¹³ C) (200 MHz) (CDCl₃ ) et par IR.
e) L'analyse élémentaire donne les résultats suivants :

| % | Calculé | Trouvé |
|---|---|---|
| C | 51,02 | 51,20 |
| H | 6,42 | 6,47 |

### EXEMPLE 2 : Utilisation du composé de formule (Ia) pour la synthèse d'un rétinoïde (Z₃₀) de formule :

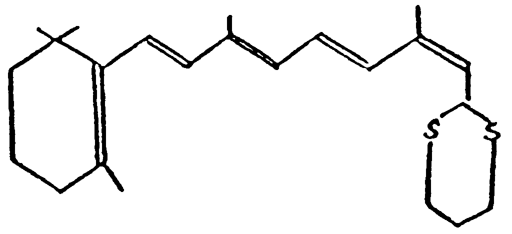

La préparation est effectuée selon le schéma réactionnel suivant :

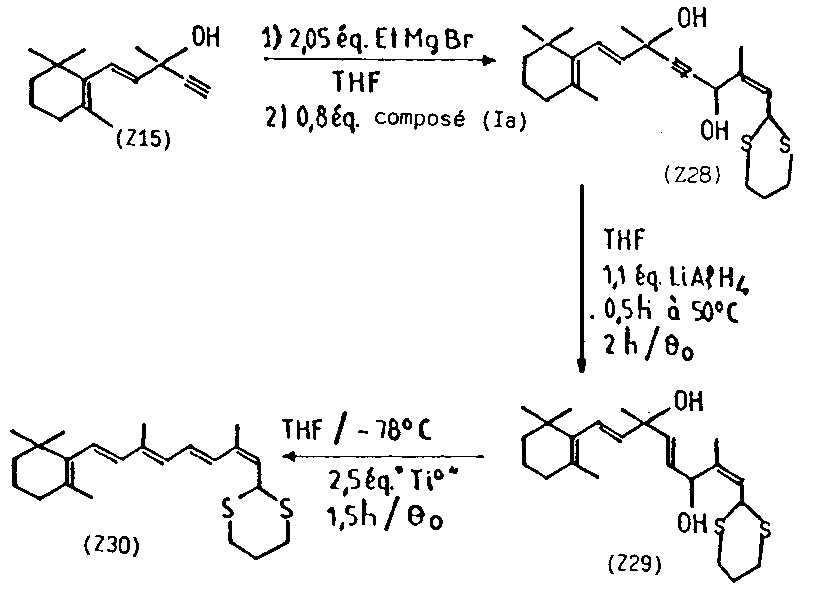

Dans ce schéma, les composés intermédiaires sont indiqués par la référence (Zᵢ), i étant un nombre entier allant de 28 à 30. θₒ désigne la température ambiante.
1ère étape : Préparation du composé (Z₂₈) :
   a) Préparation de l'éthynyl-β-ionol (Z ₁₅ ) de formule: 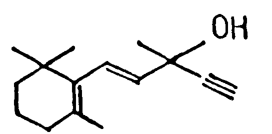
      Du magnésium sec (5,1 g - 0,210 mole) et du tétrahydrofuranne (THF) anhydre (100 ml) sont placés dans un tricol de 250 cm³ , équipé d'une ampoule à brome et d'un réfrigérant. Une solution de bromure d'éthyle (15,7 ml - 0,210 mole) dans du THF anhydre (30 ml) est alors lentement ajoutée sous argon. Après réaction du magnésium, le milieu est maintenu pendant 1 heure à température ambiante.
      D'autre part, un tricol de 500 cm³ est équipé d'un réfrigérant relié à un brûleur à huile, d'une ampoule à brome et d'un diffuseur à gaz pour acétylène (l'acétylène passant au préalable dans un piège à -78°C). L'acétylène est alors dissous pendant 1 h dans du THF anhydre (200 ml) entre -30 et -20°C. Après avoir enlevé le bain réfrigérant, on ajoute le magnésien au goutte à goutte rapide, toujours sous courant d'acétylène. La température est maintenue au-dessous de 35°C.
      Après addition du magnésien, le milieu est agite pendant 30 minutes à température ambiante. Puis de la β-ionone (21,2 ml - 0,104 mole) en solution dans du THF anhydre (20 ml) est ajoutée au goutte à goutte pendant 30 minutes. Toujours sous courant d'acétylène, la réaction est terminée au bout d'une heure et la solution jaune-verte obtenue est versée lentement sur une solution saturée en NH₄Cl (250 ml). Le milieu est extrait à l'éther (3 x 50 ml), lavé avec de l'eau (50 ml), puis avec une solution saturée en NaCl (100 ml), séché sur Na₂SO₄ et enfin concentré. Le résidu est distillé (125°C/395 Pascals) afin d'obtenir une huile incolore. On obtient 18,16 g de produit. Le compose est conservé au congélateur et à l'abri de la lumière.
      Le rendement est de: 80 %
      Les caractéristiques du produit obtenu sont les suivantes :
      a) CCM (hexane/acétate d'éthyle : 70/30) :
         Rf≃0,64.
      b) M = 218,34 g
      c) Pf = 21°C
      d) La structure a été confirmée par RMN(¹H et ¹³C) (200 MHz) (CDCl₃) et par IR.
   b) Préparation du composé (Z₂₈) de formule : 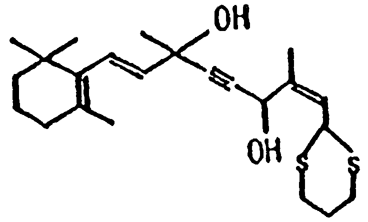
      Dans un premier temps, un réactif de Grignard est préparé à partir de 2,05 équivalents de magnésium (79,2 mg - 3,26 mmole) dans du tétrahydrofuranne (THF) anhydre (10 ml) et sous argon. 2,1 équivalents de bromoéthane (0,25 ml - 3,34 mmole) en solution dans du THF anhydre (10 ml) sont ajoutés au goutte à goutte. Dès que le magnésium est dissous, 1 équivalent du composé (Z₁₅) (348 mg - 1,59 mmole) en solution dans du THF (10 ml) est additionné lentement. Un dégagement d'éthane est observé. Après 2 h de réaction à température ambiante, 0,8 équivalent du synthon Ia (0,24 g - 1,275 mmole) en solution dans du THF (10 ml) est ajouté au milieu rapidement. Après 1,5 h, la teneur en composé (Z₂₈) n'évoluant plus comme on le constate par chromatographie en couche mince, le milieu est hydrolysé par addition lente d'une solution saturée en NH₄Cl (20 ml ; pH = 7), puis d'eau (10 ml). La phase aqueuse est extraite à l'éther (3 x 50 ml). Les phases organiques rassemblées sont lavées avec une solution saturée en NaCl (80 ml), séchées sur Na₂SO₄, puis concentrées. Le résidu est purifié par flash-chromatographie (éluant = hexane/acétate d'éthyle : 80/20). On obtient 430 mg d'une huile jaune très "mousseuse", qui peut être conservée plusieurs mois au congélateur, maie elle se dégrade très vite à température ambiante.
      Le rendement est de : 83 % par rapport au synthon Ia.
      Les caractéristiques du produit obtenu sont les suivantes :
      a) CCM (hexane/acétate d'éthyle : 70/30):
         Rf≃0,24.
      b) M = 406,66 g
      c) La structure a été confirmée par RMN(¹H) (200 MHz) (CDCl₃) et par IR.
2ème étape : Préparation du composé (Z₂₈) de formule :

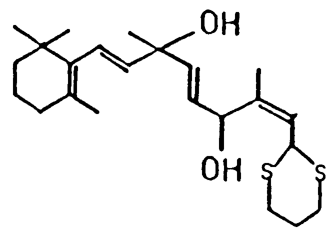

   A 1 équivalent du compose (Z₂₈) (350 mg - 0,86 mmole) en solution dans du tétrahydrofuranne (THF) anhydre (35 ml) sous argon, on ajoute goutte à goutte 1,4 équivalent de LiAlH₄ (1,1 ml de solution 0,89 M dans l'éther). Quand l'addition est terminée, le ballon réactionnel équipé d'un réfrigérant est plongé dans un bain préalablement chauffé à 50°C. A cette température, le milieu jaune fonce peu à peu. Après 1,5 h, le milieu se dégradant trop, on abaisse la température et l'agitation est poursuivie à temperature ambiante pendant 2 h. Puis le milieu est prudemment hydrolyse par addition d'une solution saturée en NH₄Cl (20 ml ; pH = 7), puis d'eau (10 ml). La phase aqueuse est extraite à l'éther (3 x 20 ml). Les phases organiques rassemblées sont lavées par une solution saturée en NaCl (50 ml), séchées sur Na₂SO₄, puis concentrées. Le résidu est purifié par flash-chromatographie (éluant = hexane/acétate d'éthyle : 90/10). On obtient 105 mg d'une huile jaune ; cette huile peut être conservée difficilement au congélateur et se dégrade très vite à température ambiante.
   Le rendement est de : 55 %
   Le produit obtenu a les caractéristiques suivantes :
   a) CCM (hexane/acétate d'éthyle : 70/30) :
      Rf ≃ 0,23.
   b) M = 408,67 g
   c) La structure a été confirmée par RMN(¹H) (200 mHz) (CDCl₃).
3ème étape : Préparation du rétinoïde (Z₃₀) de formule :

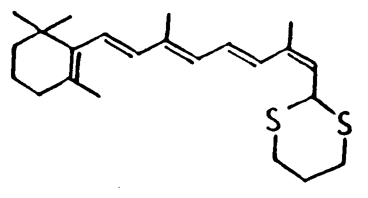

   Pour préparer le réactif contenant le titane basse valence (Ti°) le trichlorure de titane (1,5 g - 9,72 mmole) est pesé dans un ballon sec équipé d'un réfrigérant ; le ballon est purgé à l'argon et du tétrahydrofuranne (THF) anhydre (48 ml) est ajouté, suivi de 0,5 équivalent de LiAlH₄ (2,2 ml de solution 2,2M dans l'éther). Le milieu est agité pendant 10 minutes à température ambiante puis on ajoute 0,42 équivalent de triéthylamine (0,57 ml - 4,08 mmole) et le tout est chauffé au reflux pendant 1,5 h.
   A l'abri de la lumière, 2,1 équivalents du réactif obtenu (3,7 ml de la suspension) sont ajoutés, au goutte à goutte et à -78°C à 1 équivalent de composé Z₂₉ (0,14 g - 0,343 mmole) en solution dans du THF anhydre (20 ml). Le milieu est alors plongé dans un bain à 50°C et agité 0,5 h à cette température, puis hydrolysé lentement à -30°C par addition d'eau (10 ml). La phase aqueuse est extraite à l'éther (4 x 15 ml). Les phases organiques rassemblées sont filtrées sur une petite couche de célite, séchées sur Na₂SO₄, puis concentrées sous vide à température ambiante. Le résidu obtenu est purifié par flash-chromatographie (éluant = hexane/éther : 95/5). On obtient 64 mg de cristaux jaunes.
   Le rendement est de : 35 %
   Le produit obtenu a les caractéristiques suivantes :
   a) CCM (hexane/acétate d'éthyle : 70/30 :
      Rf ≃0,80.
   b) M = 374,66 g
   c) La structure a été confirmée par RMN ( ¹H) (200 MHz) (CDCl₃).

### EXEMPLE 3 : Préparation du composé de formule (Ib)

La préparation se fait selon le schéma ci-dessous.

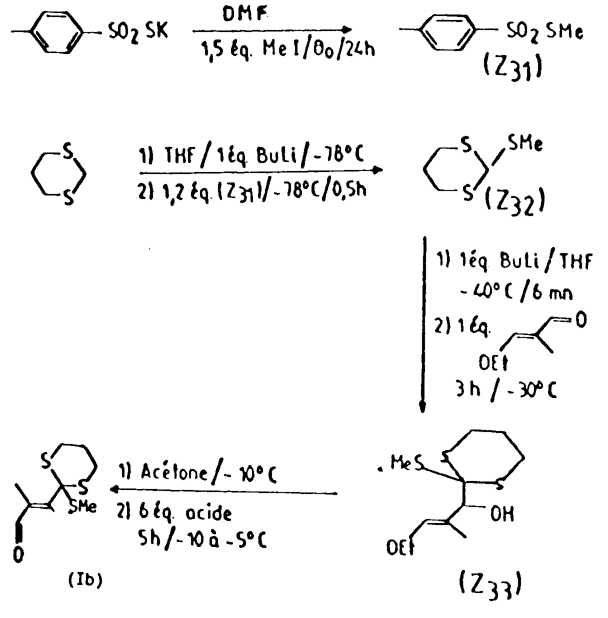

Dans ce schéma, les composés intermédiaires sont indiqués par les références (Zᵢ ), i étant un nombre entier allant de 31 à 33 ; θₒ désigne la température ambiante.
1ère étape : Préparation du S-méthyl 4-méthylbenzènethiosulfonate (Z₃₁) de formule : 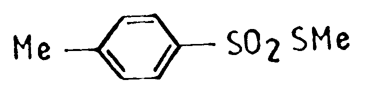 avec Me = méthyle
A 1 équivalent de toluylthiosulfonate de potassium (35 g - 0,155 mole) en solution dans le dimethylformamide distille sur tamis moléculaire 4 Angströms (500 ml), on ajoute 1,3 équivalent d'iodométhane (12,8 ml - 0,201 mole) au goutte à goutte rapide. Le milieu est agité pendant 24 h à température ambiante. En cours de réaction, il brunit peu à peu. Après dilution par addition d'eau (450 ml), le milieu est extrait au CH₂Cl₂ (5 x 150 ml). Les phases organiques rassemblées sont lavées par une solution saturée en NaHCO₃ (2 x 150 ml) et par une solution saturée en Na₂SO₃ (200 ml) ce qui permet de décolorer le milieu, puis lavées abondamment à l'eau (5 x 200 ml). Après un dernier lavage par une solution saturée en NaCl (250 ml) suivi d'un séchage sur Na ₂ SO₄ et d'une concentration, un précipité jaune est obtenu. Il est recristallisé dans de l'éther additionné d'un peu de pentane. On obtient 27,91 g de gros cristaux blancs.
Le rendement est de : 89 %
Les caractéristiques du produit obtenu sont les suivantes :
a) CCM (hexane/acétate d'éthyle : 70/30 :
   Rf≃0,5
b) Pf = 58-59°C
c) La structure a été confirmée par RMN( H¹et ¹³C) (200 MHz) (CDCl₃) et par IR.

2ème étape : Préparation du 2-méthylthio-1,3-dithiane (Z₃₂) de formule : 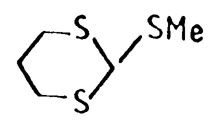 avec Me = méthyle
Une solution de 1,3-dithiane (6 g - 0,05 mole) dans du tétrahydrofuranne (THF) anhydre (200 ml) est refroidie à -78°C ; 1 équivalent de n-butyl lithium (33,3 ml de solution 1,5 M) est ajouté au goutte à goutte rapide. Le milieu est agité pendant 2 h à cette température ; puis il est ajouté à l'aide d'une canule sur 1,2 équivalent de composé (Z₃₁) (12,14 g - 0,06 mole) en solution dans du THF anhydre (80 ml) à -78°C. Lors de l'addition (qui a lieu en 35 minutes environ), un précipité blanc se forme progressivement. L'agitation est maintenue à -78°C durant 0,5 h, puis le milieu est hydrolysé par addition d'HCl 0,05 N (600 ml). Le THF est évaporé, puis le résidu est extrait par un mélange CH₂Cl₂/pentane (1/1) (4 x 200 m ). Les phases organiques rassemblées sont lavées par une solution saturée en NaHCO₃ (3 x 100 ml), par une solution saturée en NaCl (250 ml), puis séchées sur Na₂SO₄ et concentrées. Le résidu est rapidement soumis à une chromatographie sur colonne de silice (éluant = hexane/éther : 95/5) afin d'enlever l'excès de toluylthiosulfonate de méthyle (Z₃₁ ). On obtient 7,90 g d'une huile incolore qui précipite au congélateur.
Le rendement est de : 95 %
Les caractéristiques du produit obtenu sont les suivantes :
a) CCM (hexane/éther : 90/10) :
   Rf≃0,54.
b) M = 166,33 g
c) La structure a été confirmée par RMN( ¹H et ¹³C) (200 MHz) (CDCl₃).

3ème étape : Préparation du [ 3-(2-méthylthio-1,3-dithiane 2-yl) 3-hydroxy 2-méthyl 1-propenyl ] éther d'éthyle (Z₃₃) de formule : 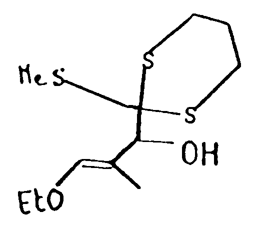
- avec: ME = méthyle
Et = éthyle

A l'orthothioester (Z₃₂ ) (7,5 g - 0,045 mole) en solution dans du tétrahydrofuranne (THF) distillé (150 ml) à -40°C, est additionné au goutte à goutte rapide 1 équivalent de n-butyl lithium (30 ml de solution 1,5M). Après 6 minutes de réaction à cette température, 1 équivalent d'éthoxyméthacroléine (5,35 ml - 0,045 mole) est ajouté. L'agitation est maintenue pendant 3 h à -30°C, puis le milieu est hydrolysé par addition d'une solution saturée en NH₄Cl (environ 200 ml - pH = 5). Dès que la température est remontée à l'ambiante, on extrait à l'éther (3 x 200 ml). Les phases organiques rassemblées sont lavées par une solution saturée en NaHCO₃ (2 x 200 ml), de l'eau (200 ml), une solution saturée en NaCl (200 ml), puis séchées sur Na₂SO₄ et concentrées. Le résidu jaune est purifie par flash - chromatographie (éluant = hexane/éther : 90/10). On obtient 10,25 g d'une huile jaune claire que l'on conserve au congélateur où elle précipite.
Le rendement est de : 81 %
Le produit obtenu a les caractéristiques suivantes :
a) CCM (hexane/éther : 90/10) :
Rf≃0,12.
b) Pf = 39-40°C
c) M = 280,48 g
d) La structure a été confirmée par RMN(¹H et ¹³C) (200 Mz) (CDCl₃)et par IR.
e) L'analyse élémentaire donne les résultats suivants :

| % | Calculé | Trouvé |
|---|---|---|
| C | 47,11 | 47,37 |
| H | 7,19 | 7,22 |

4ème étape : Préparation du composé (E) 3-(2-méthylthio-1,3-dithiane 2-yl) 2-méthylpropénal de formule 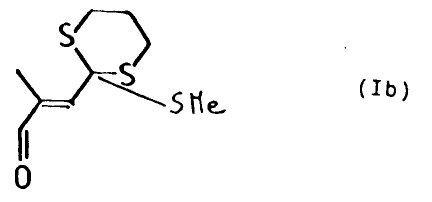 avec Me = méthyle
L'éther d'énol (Z₃₃) (9 g - 0,032 mole) est mis en solution dans de l'acétone (300 ml) à -10°C ; 6 équivalents d'acide (54 ml d'acide sulfurique à 10 % dans l'eau) sont ajoutes lentement au milieu. Celui-ci est agité pendant 5 h entre -10 et -5°C avant d'être hydrolysé par addition d'une solution saturée en NaHCO₃ (environ 200 ml ; pH = 5) et d'eau (200 ml). L'acétone est évaporée, puis le milieu est extrait à l'éther (5 x 100 ml).
Les phases organiques rassemblées sont lavées par une solution saturée en NaHCO₃ (70 ml ; pH = 7) et une solution saturée en NaCl (250 ml). Après séchage sur Na ₂ SO ₄ et concentration, le résidu est purifié par flash-chromatographie (éluant = hexane/éther : 90/10). On obtient 5,62 g d'une huile jaune.
Le rendement est de : 75 %
Les caractéristiques du produit obtenu sont les suivantes :
a) CCM (hexane/éther : 90/10) :
Rf≃0,28.
b) M = 234,41 g
c) La structure a été confirmée par RMN(¹H et ¹³C) (200 MHz) (CDCl₃) et par IR.
d) L'analyse élémentaire donne les résultats suivants :

| % | Calculé | Trouvé |
|---|---|---|
| C | 46,12 | 46,22 |
| H | 6,02 | 5,95 |

### EXEMPLE 4 : Utilisation du composé de formule (Ib) pour la synthèse du rétinoïde de formule :

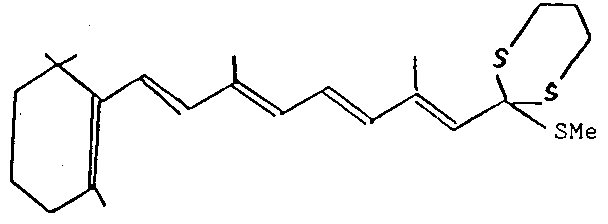

avec Me = Méthyle

La préparation se fait selon le schéma donné ci-après :

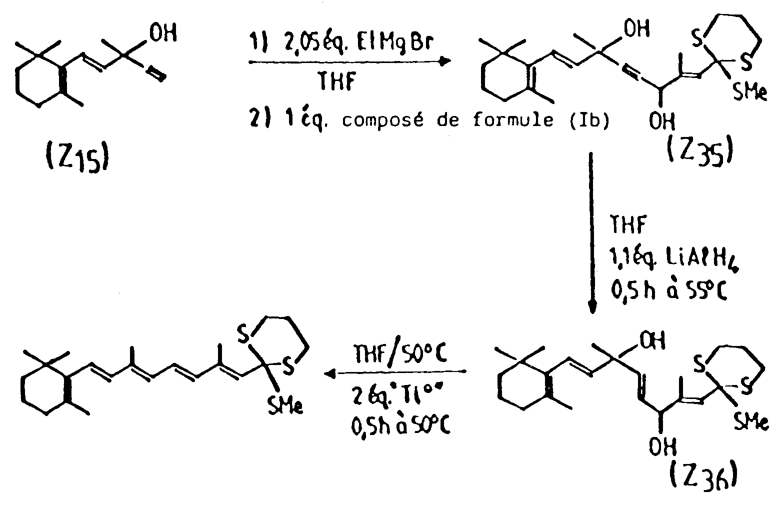

Dans ce schéma, les composés intermédiaires sont indiqués par les références (Zᵢ), i étant un nombre entier égal à 35 ou 36.

Le composé acétylénique (Z ₁₅ ) est préparé comme décrit dans l'exemple 2.
1ère étape : Préparation du composé (Z₃₅) de formule : 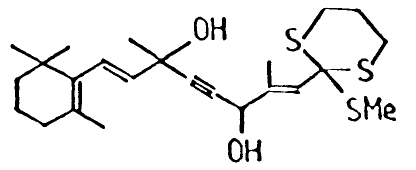 avec Me : méthyle
   Dans un premier temps, un réactif de Grignard est préparé à partir de 2,05 équivalents de magnésium (1,13 g - 0,047 mole) dans du tétrahydrofuranne (THF) anhydre (20 ml) et sous argon. On ajoute 2,1 équivalents de bromoéthane (3,6 ml - 0,048 mole) en solution dans du THF anhydre (20 ml) au goutte à goutte. Dès que tout le magnésium est dissous, 1 équivalent du composé (Z₁₅) (5 g - 0,0229 mole) en solution dans du THF (50 ml) est additionné lentement. Un dégagement d'éthane est observe. Après 1,5 h de réaction à température ambiante, 1 équivalent du composé de formule (Ib) (5,37 g - 0,0229 mole) en solution dans du THF (50 ml) est ajouté au milieu rapidement. Après 1 h la teneur en composé (Z₃₅) n'évoluant plus, comme on le constate par chromatographie en couche mince, le milieu est hydrolyse par addition lente d'une solution saturée en NH₄ Cl (50 ml), suivie d'eau (50 ml). La phase aqueuse est extraite par l'éther (3 x 80 ml). Les phases organiques rassemblées sont lavées avec une solution saturée en NaCl (100 ml), séchées sur Na₂ SO₄, puis concentrées. Le résidu est purifié par flash-chromatographie (éluant = hexane/acétate d'éthyle : 85/15). On obtient 9,33 g d'une huile jaune très "mousseuse".
   Le rendement est de : 90 %
   Le produit-obtenu a les caractéristiquee suivantes :
   a) CCM (hexane/acétate d'éthyle : 70/30) :
      Rf≃0,29.
   b) M = 452,75 g
   c) La structure a été confirmée par RMN(¹H) (200 MHz) (CDCl₃) et par IR.
2ème étape : Préparation du diol (Z₃₆) de formule : 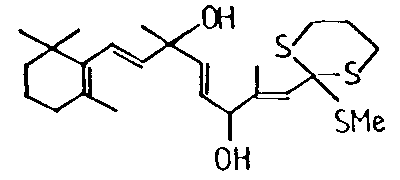 avec Me = méthyle
   A 1 équivalent du composé (Z ₃₅ ) (1,1 g - 2,43 mmole) en solution dans du tétrahydrofuranne (THF) anhydre (140 ml) sous argon est ajouté au goutte à goutte 1,1 équivalent de LiAlH₄ (2,7 ml de solution 1M dans l'éther). L'addition terminée, le ballon équipé d'un réfrigérant est plongé dans un bain préalablement chauffé à 55°C. A cette température, le milieu jaune prend peu à peu une couleur violette. Après 0,5 h, la réaction est totale et le milieu est prudemment hydrolyse par addition d'une solution saturée en NH₄Cl(60 ml), suivie d'eau (20 ml). La phase aqueuse est extraite à l'éther (3 x 70 ml). Les phases organiques rassemblées sont lavées par une solution saturée en NaCl (100 ml), séchées sur Na₂SO₄, puis concentrées. Le résidu est purifié par flash-chromatographie (éluant = hexane/acétate d'éthyle : 90/10). On obtient 0,9 g d'une huile jaune.
   Le rendement est de : 81 %
   Les caractéristiques du produit obtenu sont les suivantes :
   a) CCM (hexane/acétate d'éthyle : 70/30 :
      Rf≃0,21.
   b) M = 454,7 g
   c) La structure a été confirmée par RMN(¹H) (200 MHz) (CDCl₃).
3ème étape : Préparation du rétinoïde de formule : 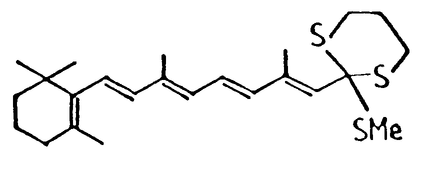 avec Me = méthyle
   Pour préparer le réactif contenant le titane basse valence (Ti°), on pèse du trichlorure de titane (0,5 g - 3,24 mmole) dans un ballon sec équipé d'un réfrigérant. Le montage est purgé à l'argon et du tétrahydrofuranne (THF) anhydre (80 ml) est ajouté. On ajoute ensuite 0,5 équivalent de LiAlH₄ (1,47 ml de solution 1,1M dans l'éther). Le milieu est agité pendant 10 minutes à température ambiante, puis 0,2 équivalent de triéthylamine (0,09 ml - 0,648 mmole) est additionné et le tout est chauffé au reflux pendant 1,5 h.
   A l'abri de la lumière, 2 équivalents du réactif contenant le titane (66,4 ml de la suspension) sont ajoutés, au goutte à goutte et à 50°C à 1 équivalent de composé (Z₃₆) (0,6 g - 1,32 mmole) en solution dans du THF anhydre (30 ml). Le milieu est agité pendant 0,5 h à cette température, puis il est hydrolysé lentement à -30°C par addition d'eau (90 ml). La phase aqueuse est extraite à l'éther (4 x 80 ml). Les phases organiques rassemblées sont filtrées sur une petite couche de célite, séchées sur Na₂SO₄, puis concentrées sous vide à température ambiante. Le résidu obtenu est purifié par flash-chromatographie (éluant = hexane/éther : 95/5). On obtient 0,82 g d'une huile jaune.
   Le rendement est de : 60 %.
   Le produit obtenu a les caractéristiques suivantes :
   a) CCM (hexane/acétate d'éthyle : 70/30) :
      Rf≃0,86.
   b) M = 420,76 g
   c) La structure a été confirmée par RMN(¹H) (200 MHz) (CDCl₃) et par IR.

## Revendications (Revendications pour l'(les) Etat(s) contractant(s) suivant(s): AT, BE, CH, DE, DK, FR, GB, GR, IE, IT, LI, LU, MC, NL, PT, SE)

1. Propénals de formule : 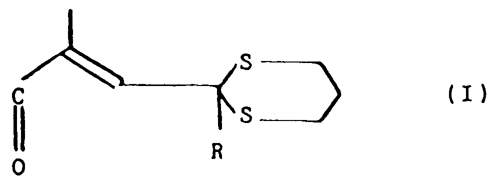 formule dans laquelle R est l'hydrogène ou un radical thioalkyle en C₁ - C₄ , le cycle dithiane étant en position cis par rapport à la fonction aldéhyde lorsque R est H et en position trans lorsque R est un radical thioalkyle.

2. Propénal selon la revendication 1 de structure cis et de formule:

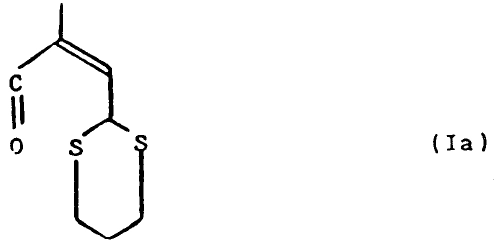

3. Propénal selon la revendication 1, de structure trans et de formule :

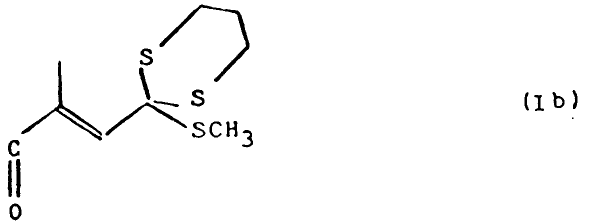

4. Procédé de préparation du propénal selon la revendication 2, caractérisé par le fait que l'on fait réagir du β-formyl méthacrylate d'éthyle de structure cis et de formule : 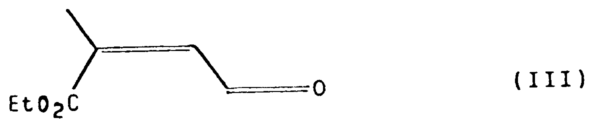 formule dans laquelle Et est un groupe éthyle, avec du 1,3-propane dithiol en présence de triflate de zinc de façon à obtenir le composé de formule : 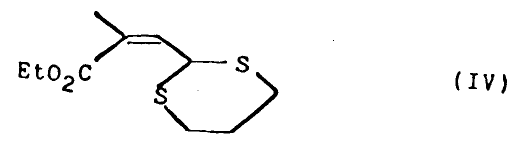 où Et a la même signification que ci-dessus indiqué, que l'on réduit ce composé de formule (IV) en présence d'hydrure de diisobutylaluminium pour obtenir le dithiane propénol de formule : 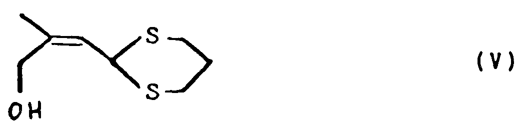 et que l'on oxyde ledit composé de formule (V) pour obtenir le cis-propénal de formule (Ia).

5. Procédé de préparation d'un propénal de structure trans selon la revendication 1, caractérisé par le fait que l'on fait réagir un alkylthiodithiane de formule : 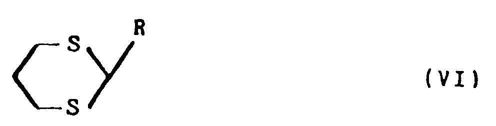 formule dans laquelle R est un radical thioalkyle en C₁ - C₄ , avec du n-butyl lithium puis avec de l'éthoxyméthacroléine de formule : 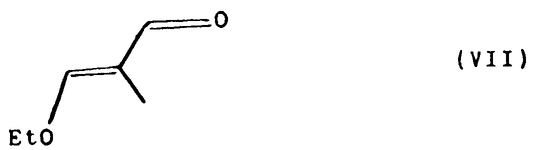 formule dans laquelle Et est un groupement éthyle, que l'on effectue une hydrolyse de façon à obtenir le composé de formule : 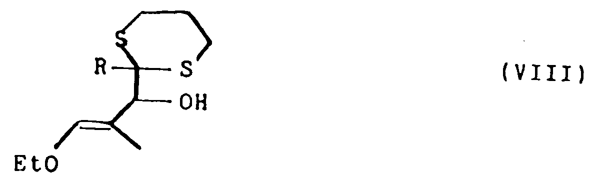 formule dans laquelle Et et R ont les significations sus-indiquées, et que l'on traite ledit composé de formule (VIII) avec de l'acétone puis avec un acide pour obtenir le composé de formule (I).

## Revendications (Revendications pour l'(les) Etat(s) contractant(s) suivant(s): ES)

1. Procédé de préparation du propénal de structure cis et de formule : 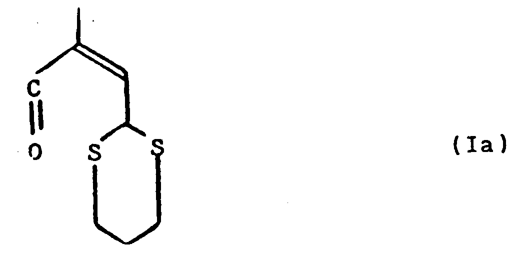 caractérisé par le fait que l'on fait réagir du β-formyl méthacrylate d'éthyle de structure cis et de formule : 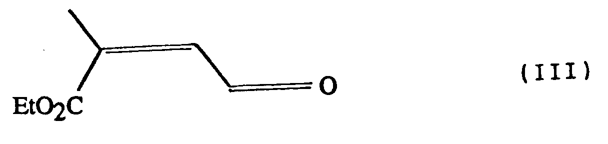 formule dans laquelle Et est un groupe éthyle, avec du 1,3-propane dithiol en présence de triflate de zinc de façon à obtenir le composé de formule : 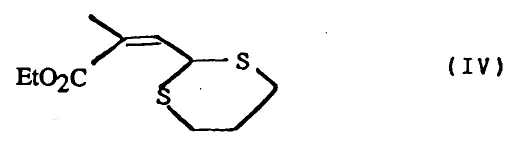 où Et a la même signification que ci-dessus indiqué, que l'on réduit ce composé de formule (IV) en présence d'hydrure de diisobutylaluminium pour obtenir le dithiane propénol de formule : 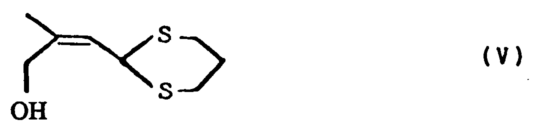 et que l'on oxyde ledit composé de formule (V) pour obtenir le cis-propénal de formule (Ia).

2. Procédé de préparation d'un propénal de structure trans de formule : 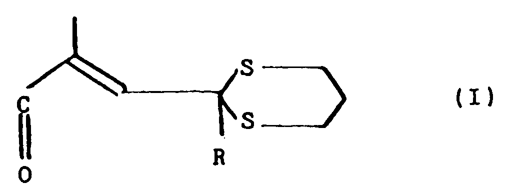 dans laquelle R est un radical thioalkyle, caractérisé par le fait que l'on fait réagir un alkylthiodithiane de formule : 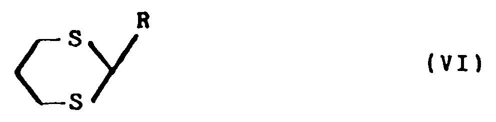 formule dans laquelle R est un radical thioalkyle en C₁-C₄, avec du n-butyl lithium puis avec de l'éthoxyméthacroléine de formule : 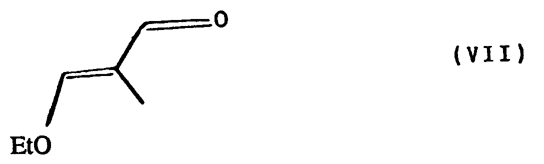 formule dans laquelle Et est un groupement éthyle, que l'on effectue une hydrolyse de façon à obtenir le composé de formule : 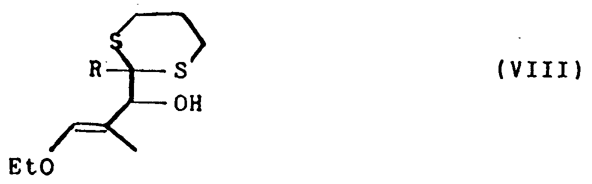 formule dans laquelle Et et R ont les significations sus-indiquées, et que l'on traite ledit composé de formule (VIII) avec de l'acétone puis avec un acide pour obtenir le composé de formule (I).

## Claims (Claims for the following Contracting State(s): AT, BE, CH, DE, DK, FR, GB, GR, IE, IT, LI, LU, MC, NL, PT, SE)

1. Propenals of formula: 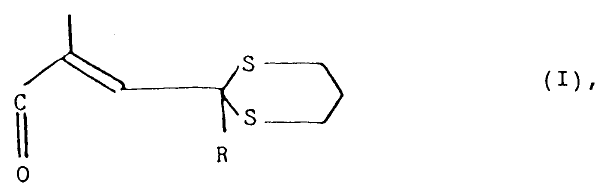 in which formula R is hydrogen or a C₁-C₄ thioalkyl radical, the dithiane ring being at the cis position with respect to the aldehyde function when R is H, and at the trans position when R is a thioalkyl radical.

2. Propenal according to Claim 1, of cis structure and of formula:

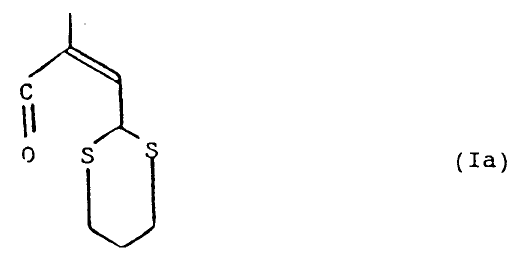

3. Propenal according to Claim 1, of trans structure and of formula:

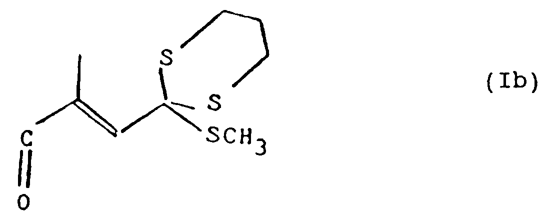

4. Process for the preparation of the propenal according to Claim 2, characterised in that ethyl β-formylmethacrylate of cis structure and of formula: 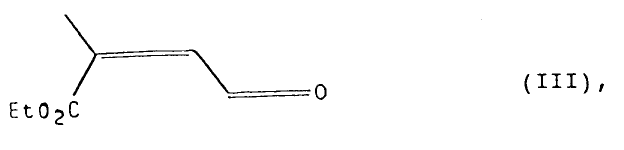 in which formula Et is an ethyl group, is reacted with 1,3-propanedithiol in the presence of zinc triflate so as to obtain the compound of formula: 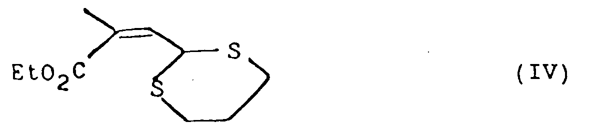 where Et has the same meaning as indicated above, in that this compound of formula (IV) is reduced in the presence of diisobutylaluminium hydride in order to obtain the dithianepropenol of formula: 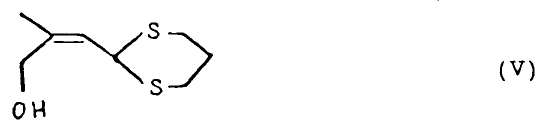 and in that the said compound of formula (V) is oxidised in order to obtain the cis-propenal of formula (Ia).

5. Process for the preparation of a propenal of trans structure according to Claim 1, characterised in that an alkylthiodithiane of formula: 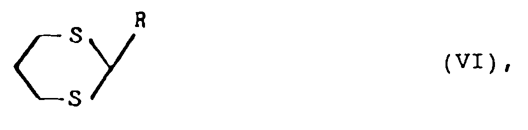 in which formula R is a C₁-C₄ thioalkyl radical, is reacted with n-butyllithium and then with ethoxymethacrolein of formula: 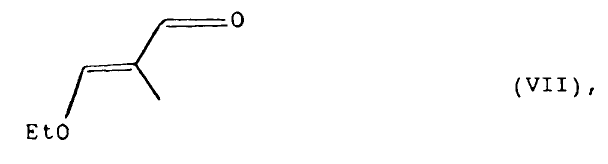 in which formula Et is an ethyl group, in that a hydrolysis is carried out so as to obtain the compound of formula: 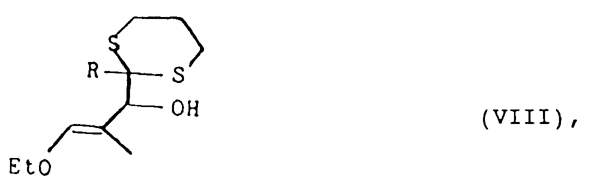 in which formula Et and R have the meanings indicated above, and in that the said compound of formula (VIII) is treated with acetone and then with an acid in order to obtain the compound of formula (I).

## Claims (Claims for the following Contracting State(s): ES)

1. Process for the preparation of propenal of cis structure having the following formula: 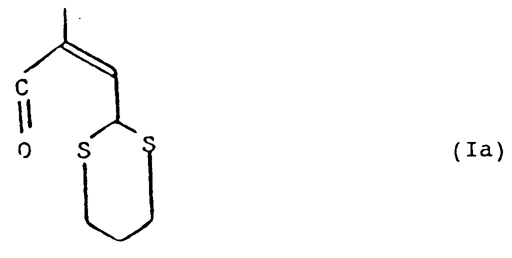 characterized in that ethyl β-formylmethacrylate of cis structure and of formula: 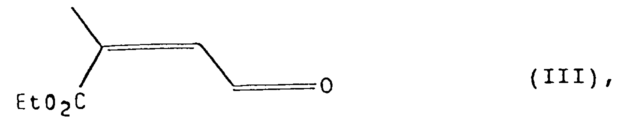 in which formula Et is an ethyl group, is reacted with 1,3-propanedithiol in the presence of zinc triflate so as to obtain the compound of formula: 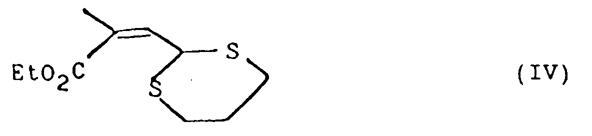 where Et has the same meaning as indicated above, in that this compound of formula (IV) is reduced in the presence of diisobutylaluminium hydride in order to obtain the dithianepropenol of formula: 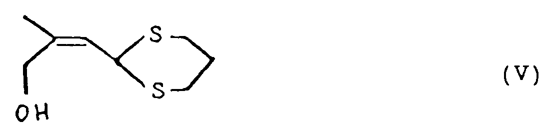 and in that the said compound of formula (V) is oxidised in order to obtain the cis-propenal of formula (Ia).

2. Process for the preparation of a propenal of trans structure having the following formula: 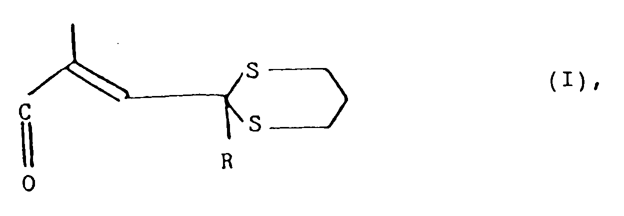 in which R is a thioalkyl radical characterized in that an alkylthiodithiane of formula: 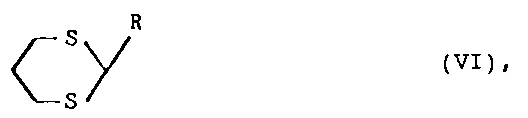 in which formula R is a C₁-C₄ thioalkyl radical, is reacted with n-butyllithium and then with ethoxymethacrolein of formula: 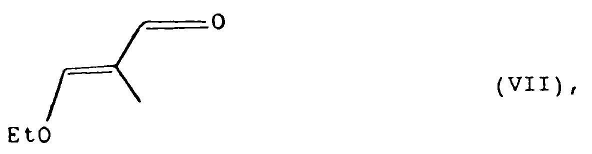 in which formula Et is an ethyl group, in that a hydrolysis is carried out so as to obtain the compound of formula: 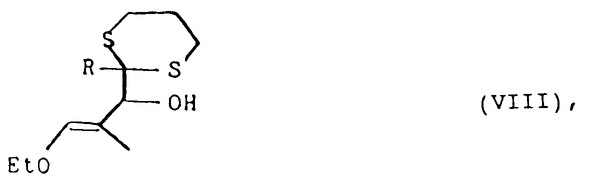 in which formula Et and R have the meanings indicated above, and in that the said compound of formula (VIII) is treated with acetone and then with an acid in order to obtain the compound of formula (I).

## Patentansprüche (Patentansprüche für folgende(n) Vertragsstaat(en): AT, BE, CH, DE, DK, FR, GB, GR, IE, IT, LI, LU, MC, NL, PT, SE)

1. Propenale der Formel 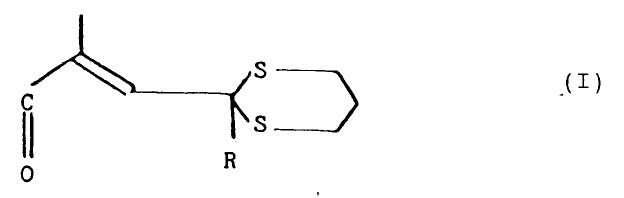 in der R Wasserstoff oder ein C₁-C₄-Thioalkyl-Rest ist, wobei der Dithian-Ring bezüglich der Aldehyd-Funktion in cis-Stellung ist, wenn R für H steht, und in trans-Stellung, wenn R ein Thioalkyl-Rest ist.

2. Propenal nach Anspruch 1 mit cis-Struktur der Formel

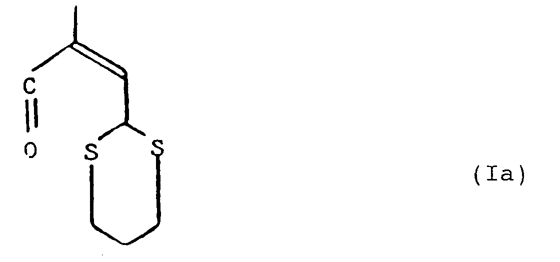

3. Propenal nach Anspruch 1 mit trans-Struktur der Formel

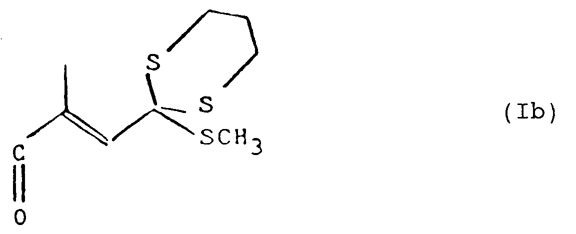

4. Verfahren zur Herstellung von Propenal nach Anspruch 2, dadurch gekennzeichnet, daß man β-Formyl-ethylmethacrylat mit cis-Struktur der Formel 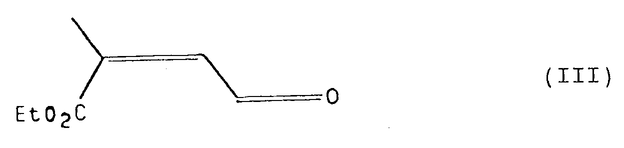 in welcher Et eine Ethylgruppe ist, mit 1,3-Propandithiol in Anwesenheit von Zinktriflat reagieren läßt, so daß man die Verbindung der Formel: 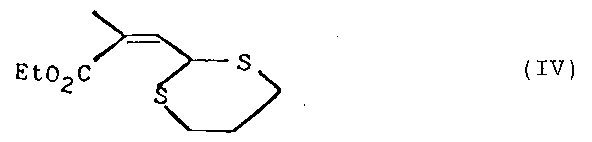 erhält, in der Et dieselbe Bedeutung wie oben hat, daß man diese Verbindung der Formel (IV) in Anwesenheit von Diisobutylaluminiumhydrid reduziert, um das Dithianpropenol der Formel 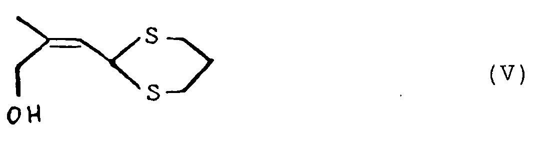 zu erhalten, und daß man diese Verbindung der Formel (V) oxidiert, um das cis-Propenal der Formel (Ia) zu erhalten.

5. Verfahren zur Herstellung eines Propenals mit trans-Struktur nach Anspruch 1, dadurch gekennzeichnet, daß man ein Alkylthiodithian der Formel 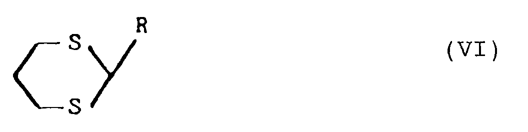 in welcher R ein C₁-C₄-Thioalkyl-Rest ist, mit n-Butyllithium und dann mit Ethoxymethacrolein der Formel 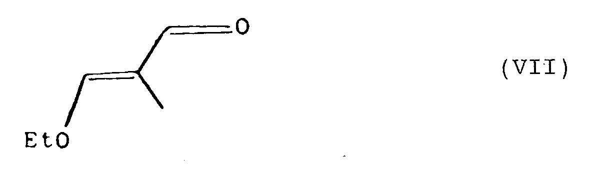 reagieren läßt, in der Et eine Ethylgruppe ist, daß man eine Hydrolyse durchführt, so daß man die Verbindung der Formel 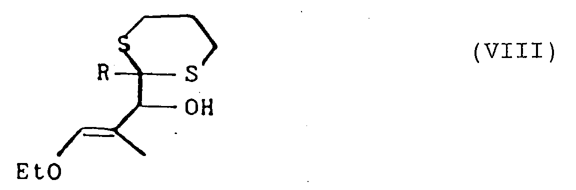 erhält, in der Et und R die oben angegebenen Bedeutungen haben, und daß man diese Verbindung der Formel (VIII) mit Aceton und dann mit einer Säure behandelt, um die Verbindung der Formel (I) zu erhalten.

## Patentansprüche (Patentansprüche für folgende(n) Vertragsstaat(en): ES)

1. Verfahren zur Herstellung des Propenals mit cis-Struktur der Formel: 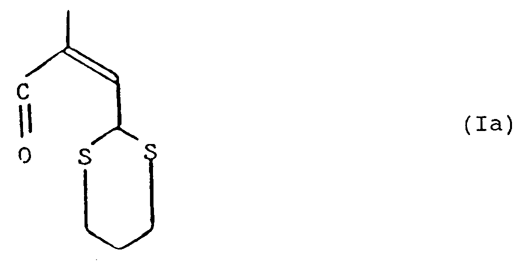 dadurch gekennzeichnet, dass man β-Formylethylmethacrylat mit cis-Struktur der Formel: 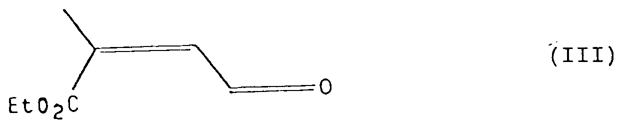 in welcher Et eine Ethylgruppe ist, mit 1,3-Propandithiol in Anwesenheit von Zinktriflat reagieren lässt, so dass man die Verbindung der Formel: 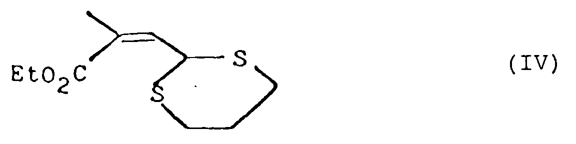 erhält, in der Et dieselbe Bedeutung wie oben hat, dass man diese Verbindung der Formel (IV) in Anwesenheit von Diisobutylaluminiumhydrid reduziert, um das Dithianpropenol der Formel: 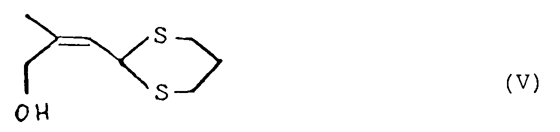 zu erhalten, und dass man diese Verbindung der Formel (V) oxidiert, um das cis-Propenal der Formel (Ia) zu erhalten.

2. Verfahren zur Herstellung des Propenals der Formel: 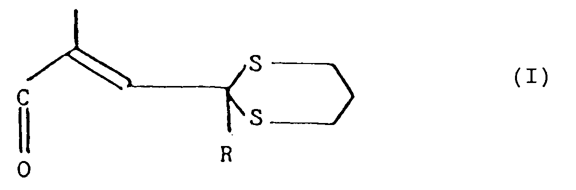 dadurch gekennzeichnet, dass man ein Alkylthiodithian der Formel: 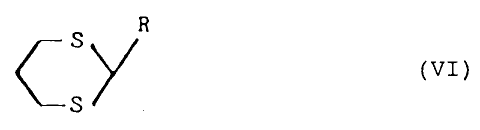 in welcher R ein C₁-C₄-Thioalkyl-Rest ist, mit n-Butyllithium und dann mit Ethoxymethacrolein der Formel 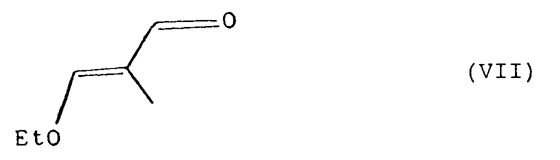 reagieren lässt, in der Et eine Ethylgruppe ist, dass man eine Hydrolyse durchführt, so dass man die Verbindung der Formel: 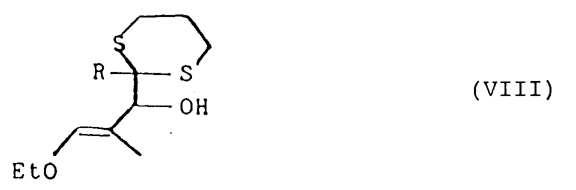 erhält, in der Et und R die oben angegebenen Bedeutungen haben, und dass man diese Verbindung der Formel (VIII) mit Aceton und dann mit einer Säure behandelt, um die Verbindung der Formel (I) zu erhalten.
